# EUROPÄISCHE PATENTSCHRIFT

(11) **EP 0 352 563 B1**
(45) Veröffentlichungstag und Bekanntmachung des Hinweises auf die Patenterteilung: **19.01.1994**
(21) Anmeldenummer: 89112889.4
(22) Anmeldetag: 14.07.1989
(51) Int. Cl.: C07C 201/12, C07C 205/37, C07C 205/25, C07C 205/26, C07C 255/53, C07C 253/30, C07D 213/69, C07D 215/20

(54) **Verfahren zur Hydroxylierung elektrophiler aromatischer Verbindungen**
Process for the hydroxylation of electrophilic aromatic compounds
Procédé d'hydroxylation de composés aromatiques électrophiles

(30) Priorität: 27.07.1988 PL 273927
(43) Veröffentlichungstag der Anmeldung: 31.01.1990
(73) Patentinhaber: BAYER AG, 51368 Leverkusen (DE)
(72) Erfinder: Makosza, Meczyslaw, Prof. Dr., PL-01-312 Warszawa (PL); Sienkiewicz, Krzysztof, PL-02-134 Warszawa (PL)

(56) Entgegenhaltungen:
- DE-A- 1 543 492
- GB-A- 1 160 077
- US-A- 3 377 386
- CHEMICAL ABSTRACTS, Band 98, 1983, Seite 621, Spalte 1, Zusammenfassung Nr.53352w, Columbus, Ohio, US; E.V. MALYKHIN et al.: "Reaction of aromatic compounds with nucleophilic reagents in a liquid amoonia medium. I. Replacement of a hydrogen atom by a hydroxy group in aromatic nitro compounds in the presence of potassium hydroxide and oxygen"

## Beschreibung

Die Erfindung betrifft ein Verfahren zur Hydroxylierung elektrophiler aromatischer Verbindungen.

Hydroxylierte elektrophile aromatische Verbindungen, z.B. Nitrophenole, Nitronaphthole und durch Nitrogruppen substituierte heteroaromatische Hydroxyverbindungen wie Nitro-hydroxy-pyridine, sind wichtige Zwischenprodukte für die Herstellung von Pflanzenschutzmitteln, Pharmazeutika und Farbstoffen. Wegen ihrer Bedeutung als Zwischenprodukte wurden bereits die verschiedensten Verfahren zur Herstellung dieser hydroxylierten elektrophilen aromatischen Verbindungen ausgearbeitet. Bislang wurden diese Verbindungen im wesentlichen durch Substituentenaustausch, z.B. durch Hydrolyse der entsprechenden Halogen-Verbindungen, durch Diazotieren der entsprechenden Aminoverbindungen und Hydrolyse der Diazoniumsalze, durch Oxidation von lithium- oder magnesiumorganischen Verbindungen der entsprechenden nitroaromatischen Verbindungen oder durch Nitrieren der entsprechenden aromatischen Hydroxyverbindungen, hergestellt.

Die GB-PS 1 160 077 beschreibt ein Verfahren zur Herstellung von Nitrophenolen durch Hydrolyse von Halogennitrobenzolen mit Alkalihydroxiden in Gegenwart von milden Oxidationsmitteln. Es handelt sich um eine nucleophile Substitution des Halogens gegen Hydroxid, wobei das Oxidationsmittel lediglich katalytisch wirkt.

Die bekannten Verfahren haben jedoch den Nachteil, daß sie nicht allgemein für die Herstellung beliebiger Nitrophenole u.s.w. sondern nur für die Herstellung ganz bestimmter Nitrophenole oder Nitronaphthole geeignet sind.

Es wurde jetzt ein allgemein für die Hydroxylierung elektrophiler aromatischer Verbindungen anwendbares Verfahren gefunden; es wurde gefunden, daß die Einführung einer Hydroxygruppe in elektrophile aromatische Verbindungen auf einfache Weise und in guten Ausbeuten gelingt, wenn man die elektrophilen aromatischen Verbindungen in Gegenwart von Basen mit organischen Hydroperoxiden umsetzt.

Die Hydroxylierung aromatischer Verbindungen mit organischen Hydroperoxiden ist bekannt (siehe z.B. die US-PSen 3 377 386, 3 553 271 und 4 182 918; die EP-A-299 893). Bislang wurden die Hydroxylierungen jedoch nur für nukleophile aromatische Verbindungen wie Benzol, Alkylbenzole, Phenole, Phenolether u.s.w. angewendet und in Gegenwart von (Lewis)Säuren und/oder chelatbildenden Verbindungen vorgenommen.

Überraschenderweise wurde gefunden, daß auch elektrophile aromatische Verbindungen durch organische Hydroperoxide hydroxylierbar werden, nämlich dann, wenn man die Umsetzung mit den Hydroperoxiden in Gegenwart von Basen vornimmt.

Gegenstand der Erfindung ist ein
Verfahren zur Hydroxylierung von elektrophilen aromatischen Verbindungen aus der Reihe
a) Nitrobenzole der Formel 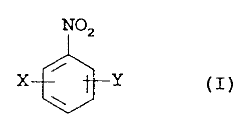 in der
   - X und Y: unabhängig voneinander für Wasserstoff, Halogen, gegebenenfalls substituiertes C₁-C₄-Alkyl, C₁-C₄-Alkoxy, C₁-C₄-Alkylmercapto, Di-C₁-C₄-alkylamino, Cyano, C₁-C₄-Alkylsulfonyl,Carboxyl, Nitro oder Hydroxy stehen, mit der Maßgabe, daß X und Y nicht gleichzeitig C₁-C₄-Alkoxy und/oder Di-C₁-C₄-Alkylamino sein dürfen und daß, wenn einer der beiden Substituenten X oder Y Hydroxy ist, der andere Substituent Y oder X Nitro sein muß;
b) 1- und 2-Nitronaphthaline;
c) eine Nitrogruppe aufweisende aromatische heterocyclische Verbindungen; und
d) Acridin, Benzthiazol und 1,2,4-Triazin
mittels Wasserstoff/Hydroxyl-Austausch durch Umsetzung mit, jeweils bezogen auf zu hydroxylierende elektrophile aromatische Verbindung, mindestens 2 Äquivalenten Base aus der Reihe Alkalihydroxid, Alkalialkoholat und Alkaliamid in Gegenwart einer mindestens äquimolaren Menge von organischem Hydroperoxid in einem organischen oder anorganischen Lösungsmittel bei Temperaturen von -30 bis +50°C.

Als Basen werden starke Basen wie Alkalihydroxide, z.B. Kaliumhydroxid, oder - vorzugsweise - Alkalialkoholate oder -amide, wie Natriummethylat, Natriumethylat, Kalium-tert.-butylat, Natriumamid oder Lithiumdiisopropylamid verwendet.

Die Basen werden in einer Menge von vorzugsweise 2 bis 7 Äquivalenten je Mol zu hydroxylierender elektrophiler aromatischer Verbindung eingesetzt.

Die organischen Hydroperoxide werden vorzugsweise in einer Menge von 1 bis 3 Mol Hydroperoxid je Mol zu hydroxylierender elektrophiler aromatischer Verbindung angewendet. Als Hydroperoxide werden die üblicherweise für die Hydroxylierung aromatischer Verbindungen verwendeten organischen Hydroperoxide verwendet; es sind dies Alkylhydroperoxide wie tert.-Butylhydroperoxid, Aralkylhydroperoxide wie α,α-Dimethylbenzylhydroperoxid (Cumylperoxid) und Acylhydroperoxide wie Formyl-, Acetyl-, Propionyl-, Butyryl-, Lauroyl- und Benzoylhydroperoxid.

Als organische Lösungsmittel kommen vor allem unpolare aprotische Lösungsmittel wie Toluol oder - vorzugsweise - polare aprotische Lösungsmittel wie Dimethylformamid, Dimethylsulfoxid, Tetrahydrofuran oder Dimethoxyethan in Betracht. Als anorganisches Lösungsmittel sei vorzugsweise flüssiges Ammoniak genannt.

Die erfindungsgemäße Hydroxylierung wird bei Temperaturen von vorzugsweise -25 bis +30°C vorgenommen.

Vertreter für die erfindungsgemäß hydroxylierbaren Nitrobenzole (I) beispielsweise Verbindungen
der Formel 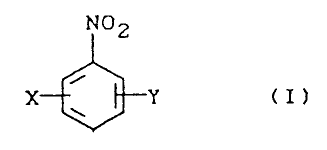
in der
- X und Y: unabhängig voneinander für Wasserstoff, Halogen, wie Fluor, Chlor, Brom und Iod, gegebenenfalls substituiertes C₁-C₄-Alkyl wie Methyl, Ethyl, Trifluormethyl, Dichlorfluormethyl; C₁-C₄-Alkoxy wie Methoxy und Ethoxy; C₁-C₄-Alkylmercapto wie Methylmercapto und Trifluormethylmercapto; Di-C₁-C₄-alkylamino wie Dimethylamino und Diethylamino; Cyano; C₁-C₄-Alkylsulfonyl wie Methylsulfonyl; Carboxyl, Nitro oder Hydroxy stehen, mit der Maßgabe, daß X und Y nicht gleichzeitig C₁-C₄-Alkoxy und/oder Di-C₁-C₄-alkylamino sein dürfen und daß wenn einer der beiden Substituenten X oder Y Hydroxy ist, der andere Substituent Y oder X Nitro sein muß;
eine Nitrogruppe aufweisende aromatische heterocyclische Verbindungen sind z.B. 2- und 3-Nitrothiophene, 2-Nitrofurane, N-alkylierte oder arylierte 2- und 3-Nitropyrrole, 2-, 3- und 4-Nitropyridine, 5-, 6- und 8-Nitrochinoline.

Das erfindungsgemäße Hydroxylierungsverfahren wird allgemein in der Weise ausgeführt, daß man die Lösung der elektrophilen aromatischen Verbindung und des organischen Hydroperoxids in dem ausgewählten Lösungsmittel unter Rühren bei Temperaturen von -30 bis +50°C mit der Lösung der Base, vorzugsweise im gleichen Lösungsmittel, versetzt. Nach beendeter Basenzugabe wird das Reaktionsgemisch noch kurze Zeit, je nach Ansatzgröße 15 bis 120 Minuten bei Raumtemperatur gerührt. Die Isolierung der aromatischen Hydroxyverbindungen aus dem Reaktionsgemisch erfolgt in Abhängigkeit von den physikalischen Eigenschaften der entstandenen aromatischen Hydroxyverbindungen in an sich bekannter Weise durch Extraktion oder Kristallisation.

Die Extraktion kann z.B. in der Weise vorgenommen werden, daß man das Reaktionsgemisch durch Einrühren in überschüssige wäßrige Mineralsäure, z.B. 1 bis 20 %ige wäßrige Salzsäure, ansäuert. Das wäßrig-saure Gemisch wird mit einem mit Wasser nicht mischbaren organischen Lösungsmittel, z.B. Methylenchlorid, die so erhaltenen Extrakte ihrerseits mit wäßriger Natronlauge, extrahiert; die vereinigten alkalischen Extrakte werden nach dem Ansäuern mit Mineralsäure abermals mit einem organischen, mit Wasser nicht mischbaren Lösungsmittel extrahiert. Nach dem Trocknen der Extrakte und Entfernen des Lösungsmittels werden die aromatischen Hydroxyverbindungen in hoher Reinheit erhalten.

Bei der erfindungsgemäßen Hydroxylierung tritt die Hydroxygruppe, sofern die ortho- oder die para-Stellung zur Nitrogruppe unsubstituiert ist, vorzugsweise in die ortho- oder para-Stellung zur Nitrogruppe ein. Die Stellung der in das aromatische System eintretenden Hydroxygruppe läßt sich durch die Reaktionsbedingungen, insbesondere durch das Lösungsmittel und die Base beeinflussen. So führt z.B. die Hydroxylierung des 1-Nitronaphthalins in Gegenwart von Kalium-tert.-butylat in Tetrahydrofuran überwiegend zum 1-Nitro-2-naphthol, während die Hydroxylierung in Gegenwart von Natriummethylat in flüssigem Ammoniak vorzugsweise 1-Nitro-4-naphthol ergibt.

### Beispiel 1

Die Lösung von 0,79 g (= 5 mMol) m-Chlornitrobenzol und 0,70 g (= 7,8 mMol) tert.-Butylhydroperoxid in 5 ml Dimethylformamid wird bei -15 bis -25°C tropfenweise unter Rühren mit der Lösung von 1,40 g (= 12,5 mMol) Kalium-tert.-butylat in 10 ml Dimethylformamid versetzt. Das Reaktionsgemisch wird abschließend 15 Minuten bei Raumtemperatur gerührt und anschließend in 60 ml 0,5 N Salzsäure eingerührt. Das wäßrige Gemisch wird mit Methylenchlorid, die vereinigten Methylenchlorid-Extrakte mit 1 N wäßriger Natriumhydroxidlösung extrahiert. Die vereinigten alkalischen Extrakte werden mit Salzsäure angesäuert und anschließend mit Methylenchlorid extrahiert. Die vereinigten Methylenchlorid-Extrakte werden nach dem Trocknen über wasserfreiem Magnesiumsulfat vom Lösungsmittel befreit.

Es werden 0,54 g (= 63 % der Theorie) 2-Chlor-4-nitro-phenol in Form eines gelben Öles erhalten.

2-Chlor-4-nitro-phenol wird in vergleichbarer Ausbeute ebenfalls erhalten, wenn als Lösungsmittel anstelle von Dimethylformamid Dimethylsulfoxid verwendet und die Reaktion bei +10°C durchgeführt wird.

### Beispiel 2

Die Lösung von 0,74 g (= 5 mMol) m-Cyanonitrobenzol und 0,70 g (= 7,8 mMol) tert.-Butylhydroperoxid in 5 ml Dimethylformamid wird wie in Beispiel 1 beschrieben mit der Lösung von 1,40 g (= 12,5 mMol) Kalium-tert.-butylat in 10 ml Dimethylformamid versetzt. Das Reaktionsgemisch wird wie in Beispiel 1 beschrieben aufgearbeitet.

Es werden 0,69 g (= 84 % der Theorie) 2-Cyano-4-nitro-phenol in Form gelber Kristalle erhalten; Fp.: 192-194°C.

### Beispiel 3

Die Lösung von 0,77 g (= 5 mMol) 3-Methoxy-5-nitropyridin und 0,70 g (= 7,8 mMol) tert.-Butylhydroperoxid in 5 ml Dimethylformamid wird unter den in Beispiel 1 beschriebenen Bedingungen mit der Lösung von 1,40 g (= 12,5 mMol) Kalium-tert.-butylat in 10 ml Dimethylformamid versetzt. Das Reaktionsgemisch wird wie in Beispiel 1 beschrieben weiterbehandelt.

Es werden 0,74 g (= 79 % der Theorie) 5-Methoxy-3-nitro-pyridon-2 (tautomere Form des 5-Methoxy-3-nitro-2-hydroxy-pyridins) in Form gelber Kristalle erhalten; Fp.: 179°C).

5-Methoxy-3-nitro-pyridon-2-wird in vergleichbarer Ausbeute ebenfalls erhalten, wenn als Base anstelle des Kalium-tert.-butylates eine äquivalente Menge Natriumethylat verwendet wird.

### Beispiel 4

Die Lösung von 1,02 g (= 5 mMol) 4-Methoxy-1-nitro-naphthalin und 0,70 g (= 7,8 mMol) tert.-Butylhydroperoxid in 5 ml Dimethylformamid wird unter den in Beispiel 1 beschriebenen Bedingungen mit der Lösung von 1,4 g (= 12,5 mMol) Kalium-tert.-butylat in 10 ml Dimethylformamid versetzt. Das Reaktionsgemisch wird wie in Beispiel 1 beschrieben weiterbehandelt.

Es werden 0,78 g (= 71 % der Theorie) 4-Methoxy-1-nitro-2-naphthol in Form gelber Kristalle erhalten; Fp.: 128-130°C.

### Beispiel 5

Die Lösung von 0,87 g (= 5 mMol) 1-Nitronaphthalin und 0,70 g (= 7,8 mMol) tert.-Butylhydroperoxid in 5 ml Tetrahydrofuran wird unter den in Beispiel 1 beschriebenen Bedingungen mit der Lösung von 1,40 g (= 12,5 mMol) Kalium-tert.-butylat in 10 ml Tetrahydrofuran versetzt. Das Reaktionsgemisch wird wie in Beispeil 1 beschrieben weiterbehandelt.

Es werden 0,74 g (= 78 % der Theorie) 1-Nitro-2-naphthol in Form gelber Kristalle erhalten; Fp.: 101-102°C.

### Beispiel 6

Die Lösung von 0,87 g (= 5 mMol) 6-Nitrochinolin und 0,70 g (= 7,8 mMol) tert.-Butylhydroperoxid in 5 ml Dimethylformamid wird bei 0°C tropfenweise unter Rühren mit der Lösung von 1,40 g (= 12,5 mMol) Kalium-tert.-butylat in 10 ml Dimethylformamid versetzt. Das Reaktionsgemisch wird 15 Minuten bei Raumtemperatur gerührt und anschließend in 60 ml 0,5 N wäßrige Salzsäure eingerührt. Der Niederschlag wird abfiltriert, mit Wasser gewaschen und getrocknet.

Es werden 0,65 g (= 65 % der Theorie) 5-Hydroxy-6-nitrochinolin in Form gelber Kristalle erhalten; Fp.: 245°C.

### Beispiel 7

Die Lösung von 8,7 g (= 50 mMol) 1-Nitronaphthalin und 8,5 g (= 0,56 mMol) Cumylhydroperoxid in 5 ml Tetrahydrofuran wird unter Rühren bei einer Temperatur von -30°C in die Lösung von 11,1 g (= 205 mMol) Natriummethylat in 200 ml flüssigem Ammoniak getropft. Nach 30-minütigem Rühren bei -30°C werden 10 g Ammoniumchlorid portionsweise eingetragen und das Ammoniak verdampft. Der Rückstand wird in 200 ml Wasser aufgenommen und die wäßrige Lösung mit Salzsäure angesäuert und mit Methylenchlorid extrahiert. Der Methylenchlorid-Extrakt wird mit 1 N wäßriger Natriumhydroxidlösung extrahiert und der alkalische Extrakt anschließend angesäuert und erneut mit Methylenchlorid extrahiert.

Nach dem Trocknen der Lösung und Einengen im Vakuum werden 8,4 g (= 87 % der Theorie) 4-Nitro-1-naphthol in Form gelber Kristalle erhalten; Fp.: 166-169°C.

## Patentansprüche

1. Verfahren zur Hydroxylierung von elektrophilen aromatischen Verbindungen aus der Reihe
a) Nitrobenzole der Formel 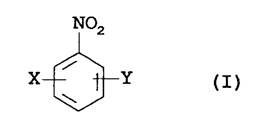 in der
X und Y unabhängig voneinander für Wasserstoff, Halogen, gegebenenfalls substituiertes C₁-C₄-Alkyl, C₁-C₄-Alkoxy, C₁-C₄-Alkylmercapto, Di-C₁-C₄-alkylamino, Cyano, C₁-C₄-Alkylsulfonyl,Carboxyl, Nitro oder Hydroxy stehen, mit der Maßgabe, daß X und Y nicht gleichzeitig C₁-C₄-Alkoxy und/oder Di-C₁-C₄-Alkylamino sein dürfen und daß, wenn einer der beiden Substituenten X oder Y Hydroxy ist, der andere Substituent Y oder X Nitro sein muß;
b) 1- und 2-Nitronaphthaline;
c) eine Nitrogruppe aufweisende aromatische heterocyclische Verbindungen; und
d) Acridin, Benzthiazol und 1,2,4-Triazin
mittels Wasserstoff/Hydroxyl-Austausch durch Umsetzung mit, jeweils bezogen auf zu hydroxylierende elektrophile aromatische Verbindung, mindestens 2 Äquivalenten Base aus der Reihe Alkalihydroxid, Alkalialkohol und Alkaliamid in Gegenwart einer mindestens äquimolaren Menge von organischem Hydroperoxid in einem organischen oder anorganischen Lösungsmittel bei Temperaturen von -30 bis +50°C.

2. Verfahren nach Anspruch 1, dadurch gekennzeichnet, daß man die Base in einer Menge von 2 bis 7 Äquivalenten je Mol zu hydroxylierender elektrophiler aromatischer Verbindung einsetzt.

3. Verfahren nach Anspruch 1 oder 2, dadurch gekennzeichnet, daß man die Hydroxylierung in organischen polaren aprotischen Lösungsmitteln vornimmt.

## Claims

1. Process for the hydroxylation of electrophilic aromatic compounds from the series consisting of
a) nitrobenzenes of the formula 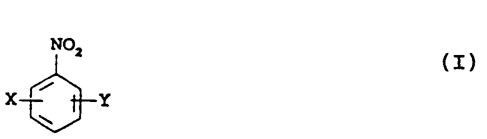 in which
X and Y, independently of one another, represent hydrogen, halogen, substituted or unsubstituted C₁-C₄-alkyl, C₁-C₄-alkoxy, C₁-C₄-alkylmercapto, di-C₁-C₄-alkylamino, cyano, C₁-C₄-alkylsulphonyl, carboxyl, nitro or hydroxyl, with the proviso that X and Y must not simultaneously be C₁-C₄-alkoxy and/or di-C₁-C₄-alkylamino and that, if one of the two substituents X or Y is hydroxyl, the other substituent Y or X must be nitro;
b) 1- and 2-nitronaphthalenes;
c) nitro-containing aromatic heterocyclic compounds; and
d) acridine, benzothiazole and 1,2,4-triazine by means of hydrogen/hydroxyl exchange by reaction with, in each case relative to the electrophilic aromatic compound to be hydroxylated, at least 2 equivalents of base from the series consisting of alkali metal hydroxide, alkali metal alcoholate and alkali metal amide, in the presence of an at least equimolar amount of organic hydroperoxide in an organic or inorganic solvent at temperatures of -30 to +50°C.

2. Process according to Claim 1, characterized in that the base is used in an amount of 2 to 7 equivalents per mole of electrophilic aromatic compound to be hydroxylated.

3. Process according to Claim 1 or 2, characterized in that hydroxylation is carried out in organic polar aprotic solvents.

## Revendications

1. Procédé d'hydroxylation de composés aromatiques électrophiles choisis dans la série suivante:
a) nitrobenzènes de formule 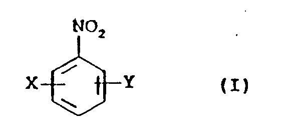 dans laquelle X et Y représentent indépendamment l'un de l'autre un atome d'hydrogène ou d'halogène, un groupe alkyle en C₁-C₄ éventuellement substitué, un alcoxy en C₁-C₄, un alkylmercapto en C₁-C₄, un dialkylamino en C₁-C₄, un cyano, un alkylsulfonyle en C₁-C₄, un carboxyle, un nitro ou un hydroxyle avec, pour condition que X et Y ne peuvent pas représenter simultanément un alcoxy en C₁-C₄ et/ou un dialkylamino en C₁-C₄ et que, si l'un des substituants X ou Y est un radical hydroxyle, l'autre substituant Y ou X doit être un groupe nitro;
b) les 1- et 2-nitronaphtalènes;
c) des composés hétérocycliques aromatiques présentant un groupe nitro; et
d) l'acridine, le benzothiazole et la 1,2,4-triazine
par échange hydrogène/hydroxyle en faisant réagir au moins deux équivalents de base rapportés au composé aromatique électrophile à hydroxyler, choisis dans la série des hydroxydes alcalins, des alcoolates alcalins et des amidures alcalins en présence d'une quantité au moins équimolaire d'hydroperoxyde organique dans un solvant organique ou inorganique, à des températures comprises entre -30 et +50°C.

2. Procédé selon la revendication 1, caractérisé en ce que l'on utilise la base en quantités comprises entre 2 et 7 équivalents par mole de composés aromatiques électrophiles à hydroxyler.

3. Procédé selon la revendication 1 ou 2, caractérisé en ce que l'on effectue l'hydroxylation dans des solvants organiques aprotiques polaires.
